# EUROPÄISCHE PATENTSCHRIFT

(11) **EP 2 785 323 B1**
(45) Veröffentlichungstag und Bekanntmachung des Hinweises auf die Patenterteilung: **18.07.2018**
(21) Anmeldenummer: 12805908.6
(22) Anmeldetag: 26.11.2012
(51) Int. Cl.: A61K 8/368, A61K 8/49, A61K 8/19, A61Q 5/10, A61K 8/9789

(54) **MITTEL UND VERFAHREN ZUR FäRBUNG VON KERATINFASERN**
AGENT AND METHOD FOR COLOURING KERATIN FIBRES
AGENTS ET PROCÉDÉ POUR TEINDRE LES FIBRES KÉRATINIQUES

(30) Priorität: 28.11.2011 AT 17522011
(43) Veröffentlichungstag der Anmeldung: 08.10.2014
(73) Patentinhaber: GW Cosmetics GmbH, 2333 Leopoldsdorf (AT)
(72) Erfinder: MARTON, Istvan, A-1090 Wien (AT)
(74) Vertreter: Sonn & Partner Patentanwälte
(86) Internationale Anmeldenummer: PCT/AT2012/050184
(87) Internationale Veröffentlichungsnummer: WO 2013/078492

(56) Entgegenhaltungen:
- EP-A2- 0 327 345
- EP-A2- 2 196 180
- DE-U1-202004 016 367
- FR-A1- 2 951 940
- US-A- 35 840
- US-A- 40 918
- US-A- 6 099 591

## Beschreibung

Die vorliegende Erfindung betrifft eine Zusammensetzung zum permanenten Färben von Keratinfasern, insbesondere zum permanenten Färben von Wimpern und Augenbrauen.

Gemäß Wilfried Umbach, Kosmetik - Entwicklung, Herstellung und Anwendung kosmetischer Mittel, 2. Auflage, Georg Thieme Verlag, Stuttgart, 1995, lässt sich die Haarfärbung in Bezug auf die Zahl der möglichen Haarwäschen nach der Färbung und der jeweiligen Haltbarkeit der Farbänderung in temporäre, semipermanente und permanente Färbungen einteilen. Eine temporäre Haarfärbung verschwindet nach dem ersten Waschen mit einem Haarshampoo, sie basiert auf einer physikalischen Haftung eines Farbstoffes. Eine semipermanente Haarfärbung hält circa zwei bis zehn Haarwäschen aus und eine permanente Färbung übersteht mehr als zehn Haarwäschen. Bei der permanenten Färbung werden Farbstoffe chemisch an bestimmte Aminosäuren im Haar gekoppelt oder beim Blondieren Pigmente des Haares oxidiert.

Für die permanente Färbung von Augenbrauen und Wimpern wurden bisher Oxidationsfarbstoffe verwendet, die unmittelbar vor der Anwendung mit Wasserstoffperoxyd vermischt wurden. Die Färbung entstand dabei durch Oxidation einer Mischung bestimmter Entwickler- und Kupplersubstanzen in Gegenwart des Wasserstoffperoxyds.

Aus der DE 298 05 893 ist ein Mittel für die oxidative Färbung von Haaren bekannt, das unmittelbar vor der Anwendung durch Vermischen einer Entwicklerlotion mit einer Farbmasse vermischt wird und diese auf einen sauren pH-Wert eingestellt ist. Durch ein solches oxidatives Färben von Haaren lässt sich zwar ein Haarfärbemittel bereitstellen, das einen pH-Wert zwischen 5 und 7 besitzt, allerdings ist das Herstellungsverfahren relativ aufwendig und auch die Einwirkzeit ist vergleichsweise lange, sodass das Haarfärbemittel sich nur schlecht zum Färben von Wimpern oder Augenbrauen eignet. Ein solches oxidatives Färben mit Wasserstoffperoxid und Farbstoffen ist zur Zeit nur gewerblich anwendbar und wird möglicherweise überhaupt verboten werden.

Ferner ist aus der DE 203 14 464 eine Kosmetikzubereitung enthaltend wenigstens ein Färbemittel, welches in der Zubereitung unlöslich ist und unter UV-Licht Fluoreszenzeffekte ergibt, bekannt, mit welcher Zubereitung Teile der Haut oder Haare dekorativ gefärbt werden können. Solche Mittel besitzen jedoch nur eine begrenzte Haltbarkeit und müssen daher häufig erneuert werden, was als aufwendig empfunden wird.

In der AU-B-61430/80 wird weiters ein Mittel zum Färben von Haaren gezeigt, welches ein Färbemittel, Ammoniak, Alkohol, Tanninsäure und ein Shampoo umfasst. Das Mittel zum Färben von Haaren wird dabei auf die Haare aufgetragen, um die gewünschte Einfärbung zu erhalten.

US 3 194 734 offenbart Haarfärbezusammensetzungen, die Ammoniak und Dihydroxy-5,6-indole zusammen mit einem Oxidationsmittel, wie Wasserstoffperoxyd, enthalten.

Gemäß der DE 20 2004 016 367 U1 umfasst ein Mittel zum Färben von Haaren eine wässrige Lösung mit Tannin (Gerbsäure) und Ammoniak. Durch dieses Mittel werden die Haare aufgeweicht und können dann in einem zweistufigen Prozess z.B. mit Silbernitratgel oder anderen Färbemitteln gefärbt werden.

Die DE 28 06 603 A1 offenbart Mittel zur Färbung von Augenbrauen, Wimpern und Barthaaren, bestehend aus zwei voneinander getrennten wässrigen Lösungen, von denen die eine Lösung Brenzcatechin und die andere Lösung Silbernitrat enthält. Bei der Silbernitratlösung kann es sich auch um eine ammoniakalische Lösung handeln.

H. S. Redgrove and G. A. Foan, Hair-dyes and Hair-dyeing Chemistry and Technique 1934, Chapter V: Silver dyes, pg. 35-42 and Chapter VI Tannin - Rasticks, pg. 43-47 offenbart eine Formulierung "B" enthaltend Pyrogallol sowie ein Silbersalz in Form einer ammoniakalischen Lösung.

Auch beispielsweise aus Ullmanns Enzyklopädie der technischen Chemie, 3. Auflage, Band 10, S. 734, ist die Verwendung von anorganischen Silbernitratlösungen beziehungsweise anderen Metallsalzen, gemischt mit Pyrogallol-Lösung als Färbemittel, bekannt. Dabei reagierten die Silberionen mit der Aminosäure Cystein im Haar, wodurch Farbtöne zwischen Blond und Schwarz eingestellt werden können.

Andererseits werden zum Nuancieren von Keratinfasern, also für die semipermanente Färbung, Farbstoffe verwendet, welche eine hohe Affinität zum Keratin des Haares haben, wobei die Bindung dieser Farbstoffe physikalisch erfolgt. Als synthetische semipermanente Haarfärbemittel werden Nitrophenyldiamine, Azo- und Chinonimin-Farbstoffe in Verbindung mit organischen Lösungsvermittlern wie Glycolether oder Polypropylen verwendet, die natürlichen semipermanenten Haarfärbemittel Henna (Blätter und Stängel von Lawsonia alba Lam. oder L. inermis L., Reng (aus der Indigopflanze), Kamille (Apigenin), Holz- und Rindenextrakte und Rastik (Pyrogallol und Eisen- bzw. Kupfersalze) haben heute an Bedeutung verloren.

Aufgabe der Erfindung ist es, eine von Wasserstoffperoxyd freie Zusammensetzung zum permanenten Färben von Keratinfasern, insbesondere zum permanenten Färben von Wimpern und Augenbrauen, zu schaffen, welche gleichbleibende Farbqualität und Dauerhaftigkeit der Färbung der Augenbrauen und Wimpern gewährleistet.

Diese Aufgabe wird erfindungsgemäß dadurch gelöst, dass eine gebrauchsfertige Färbezusammensetzung vorgesehen wird, enthaltend eine Komponente A umfassend mindestens ein Extrakt von Zaubernuss, Grüntee, dem Holz der Gerber-Akazie Acacia catechu, Granatapfel und Mischungen hiervon, zusammen mit einer Komponente B umfassend ein Silbersalz in Form einer ammoniakalischen Lösung, wobei die gebrauchsfertige Färbezusammensetzung aus dem enthaltenen Extrakt mindestens 0,02 Gew.% mindestens einer Verbindung, ausgewählt aus der Gruppe bestehend aus Catechine, Gallussäure, Gallate und Mischungen hievon, enthält. Gallate sind Derivate der Gallussäure und kommen z.B. in Eichenrinde und Galläpfeln sowie Grüntee und Oolong vor. Zusätzlich können auch synthetische Gallate zum Einsatz kommen, bevorzugt werden Methylgallat, Ethylgallat, Propylgallat (E 310), Octylgallat (E 311) und Dodecylgallat (E 312) und Mischungen hievon. Besonders bevorzugt wird Propylgallat. Die gebrauchsfertige Färbezusammensetzung kann durch Vermischen von Einzelkomponenten, umfassend mindestens eine Verbindung, ausgewählt aus der Gruppe bestehend aus Catechine, Gallussäure, Gallate und Mischungen hievon (Komponente A) einerseits und umfassend ein Silbersalz (Komponente B) andererseits, vor der Anwendung hergestellt werden, oder es können die einzelnen Komponenten in beliebiger Reihenfolge auf die Augenbrauen und Wimpern aufgetragen werden. Dabei können die einzelnen Komponenten in flüssiger, cremiger oder in Gelform vorliegen. Zum Verkauf können die Inhaltsstoffe der Komponente A auch in Pulverform vorliegen, welches Pulver vor dem Aufbringen oder Mischen durch Auflösen bzw. Dispergieren in ein oder mehreren geeigneten Lösungs- bzw. Trägermittel, z.B. Wasser, gebrauchsfertig zubereitet werden.

Vorzugsweise enthält gemäß der vorliegenden Erfindung die gebrauchsfertige Färbezusammensetzung Silbernitrat als Silbersalz, besonders bevorzugt liegt dabei das Silbernitrat zumindest in Form der Komponente B in Gelform vor.

Gemäß einer weiteren bevorzugten Ausführungsform der vorliegenden Erfindung enthält die gebrauchsfertige Färbezusammensetzung weiters mindestens eine Verbindung ausgewählt aus der Gruppe umfassend p-Phenylendiamin, p-Toluylendiamin, N-Phenyl-p-phenylendiamin, Hydroxyethyl-p-phenylendiamin, N,N'-Bis-hydroxyethyl-p-phenylendiamin, 2-Chloro-p-phenylendiamin, Triaminopyrimidin, Tetraaminopyrimidin, m-Aminophenol, p-Aminophenol, o-Aminophenol, Resorcinol, 2-Methylresorcinol, 4-Chlororesorcinol, 2,6-Diaminopyridin, 2,6-Dimethoxy-3,5-diaminopyridin, 2-Amino-3-hydroxypyridin, 2-Methyl-5-hydroxyethylaminophenol, NN'Bis-Hydroxyethyl-p-phenylendiamin 2,4-Diaminophenoxyethanol, 2-Amino-4-Hydroxyethylaminoanisolsulfat, 4-Amino-m-Cresol, 6-Amino-m-Cresol, Phenylmethylpyrazolon, 4-Amino-2-hydroxytoluol, p-Methylaminophenol, Hydroxybenzomorpholin, 1,3-bis-(2,4-Diaminophenoxy)propan, 6-Methoxy-2-methylamino-3-aminopyridin, 5-Amino-4-chloro-o-Cresol, 3-Amino-2,4-dichlorophenol, 1-Naphtol, 1,5-Naphtalendiol, 2,7-Naphtalendiol, 1-Hydroxyethyl-4,5-diaminopyrazolsulfat, 2,2'-Methylenbis-4-aminophenol, 2-Methyl-1-naphtol, 1-Acetoxy-2-methoxynaphtalen und Mischungen hievon.

Der pH-Wert der gebrauchsfertigen Färbezusammensetzung liegt im Bereich von etwa 5 bis 9, vorzugsweise zwischen etwa 6 und 8. Die gebrauchsfertige Färbezusammensetzung kann weitere Zusätze, wie Pigmente, Tenside, Verdickungsmittel, Emulsionsgrundlagen, Lösungsmittel, Konservierungsmittel sowie Natriumsulfit, enthalten.

Gemäß einer bevorzugten Ausführungsform der vorliegenden Erfindung umfasst die gebrauchsfertige Färbezusammensetzung als Komponente A Extrakte von Zaubernuss, Grüntee, Akazie und Granatapfel sowie Mischungen hievon, enthaltend Gallate, vorzugsweise in einer Menge von 0,1 bis 5 Gew.%, besonders bevorzugt 0,3 bis 3 Gew.% wobei die Angaben an Gew.% sich auf die Komponente A beziehen.

Erfindungsgemäß vorgesehen ist auch ein Erzeugnis zur gleichzeitigen oder zeitlich abgestuften Anwendung, umfassend zwei Komponenten A und B, wobei Komponente A Extrakte von Zaubernuss, Grüntee, Akazie und Granatapfel sowie Mischungen hievon, enthaltend mindestens 0,02 Gew.% Gallate, vorzugsweise in einer Menge von 0,1 bis 5 Gew.%, besonders bevorzugt 0,3 bis 3 Gew.% umfasst, wobei die Angaben an Gew.% sich auf Komponente A beziehen, und Komponente B eine Zubereitung enthaltend Silbernitrat in einer basischen ammoniakalischen Lösung mit einem pH-Wert von zwischen 7 und 10, bevorzugt 8 bis 9, umfasst. Die beiden Komponenten können dabei jeweils in Form einer wässrigen Lösung, einer Emulsion, oder eines Gels vorliegen, bzw. können die Inhaltsstoffe der Komponente A auch in Pulverform vorliegen, welches Pulver vor dem Aufbringen oder Mischen durch Auflösen bzw. Dispergieren in ein oder mehreren geeigneten Lösungs- bzw. Trägermittel, z.B. Wasser, gebrauchsfertig zubereitet werden, die Komponenten A und B können zur Stabilisierung auch noch Verdickungsmittel oder andere Zusätze enthalten.

Vorzugsweise enthält die Komponente B Silbernitrat in einer Menge von 2 bis 3 Gew.%, bevorzugt 1,8 bis 2,2 Gew.%, wobei die Angaben an Gew.% sich auf Komponente B beziehen.

Die gebrauchsfertige Färbezusammensetzung wird vorteilhafterweise nach Aufbringung, wie erwähnt entweder nach Vermischen der Komponenten und anschließendem Aufbringen oder nach Aufbringen der einzelnen Komponenten getrennt voneinander in beliebiger Reihenfolge, in einer Gesamtzeit von 3 bis 30 Min., bevorzugt zwischen 10 bis 20 Min. einwirken gelassen.

Die vorliegende Erfindung wird nun unter Bezugnahme auf die nachfolgenden Beispiele, auf welche sie jedoch nicht beschränkt sein soll, näher erläutert.

In den Beispielen wurden die Extrakte bzw. Verbindungen der folgenden Hersteller verwendet:

| | |
|---|---|
| Acacia catechu Extrakt | Acacia catechu BE 3% Catechins, Denk Ingredients GmbH, München (DE) |
| Grüntee-Extrakt I | Green Tea Dry Extract 90, Biopole, Clermont-Limagne (FR) |
| Grüntee-Extrakt II | Green Tea Extract 50% Polyphenols, Denk Ingredients GmbH, München (DE) |
| Zaubernuss-Extrakt | Hamamelis Leaves Extract, Denk Ingredients GmbH, München (DE) |
| Granatapfel-Extrakt | Pomgranate Extract WS, Denk Ingredients GmbH, München (DE) |
| Gallussäure | Orion Chemicals Metalchem Spain, Barcelona (ES) |
| Propylgallat | Syntharo Fine Chemicals, Troisdorf (DE) |
| Ethylgallat | Sigma-Aldrich Produktion GmbH, Buchs (CH) |

### Beispiel 1.

**Farbe (Komponente A)**

| | |
|---|---|
| Propylgallat (E 310) | 0,3 Gew.% |
| Acacia catechu Extrakt | 0,4 Gew.% |
| Zaubernuss-Extrakt | 0,2 Gew.% |
| Ammonium Acryloyldimethyltaurat/VP Copolymer | 1,0 Gew.% |
| Entmineralisiertes Wasser auf | 100,0% Gew.% |

### Beispiel 2.

**Farbe (Komponente A)**

| | |
|---|---|
| Grüntee-Extrakt I | 3,0 Gew.% |
| Ethylgallat | 0,8 Gew.% |
| Ammonium Acryloyldimethyltaurat/VP Copolymer | 1,0 Gew.% |
| Entmineralisiertes Wasser auf | 100,0% Gew.% |

### Beispiel 3.

**Farbe (Komponente A)**

| | |
|---|---|
| Zaubernuss-Extrakt | 2,0 Gew.% |
| Ammonium Acryloyldimethyltaurat/VP Copolymer | 0,8 Gew.% |
| p-Phenylendiamin | 0,3 Gew.% |
| Gallussäure | 0,2 Gew.% |
| Entmineralisiertes Wasser auf | 100,0% Gew.% |

### Beispiel 4.

**Farbe (Komponente A)**

| | |
|---|---|
| Zaubernuss-Extrakt | 0,5 Gew.% |
| Grüntee-Extrakt II | 0,5 Gew.% |
| Granatapfel-Extrakt | 0,5 Gew.% |
| Acacia catechu Extrakt | 0,5 Gew.% |
| Ammonium Acryloyldimethyltaurat/VP Copolymer | 1,0 Gew.% |
| Entmineralisiertes Wasser auf | 100,0% Gew.% |

### Beispiel 5.

**Farbe (Komponente A)**

| | |
|---|---|
| Zaubernuss-Extrakt | 10,0 Gew.% |
| Ammonium Acryloyldimethyltaurat/VP Copolymer | 0,9 Gew.% |
| Entmineralisiertes Wasser auf | 100,0% Gew.% |

### Beispiel 6.

**Silbernitratgel (Komponente B)**

| | |
|---|---|
| Silbernitrat | 3,0 Gew.% |
| Carbomer | 0,7 Gew.% |
| Ammoniak 25%-ig | 3,5 Gew.% |
| Entmineralisiertes Wasser auf | 100,0 Gew.% |

### Beispiel 7.

**Silbernitratgel (Komponente B)**

| | |
|---|---|
| Silbernitrat | 2,2 Gew.% |
| Carbomer | 0,7 Gew.% |
| Ammoniak 25%-ig | 3,5 Gew.% |
| Entmineralisiertes Wasser auf | 100,0 Gew.% |

### Beispiel 8.

**Silbernitratgel (Komponente B)**

| | |
|---|---|
| Silbernitrat | 1,8 Gew.% |
| Carbomer | 0,7 Gew.% |
| Ammoniak 25%-ig | 3,5 Gew.% |
| Entmineralisiertes Wasser auf | 100,0 Gew.% |

### Beispiel 9.

**Silbernitratgel (Komponente B)**

| | |
|---|---|
| Silbernitrat | 1,0 Gew.% |
| Carbomer | 0,7 Gew.% |
| Ammoniak 25%-ig | 3,5 Gew.% |
| Entmineralisiertes Wasser auf | 100,0 Gew.% |

Die Komponenten A und B wurden nach Aufbringen getrennt voneinander (zuerst Aufbringen von Komponente A in etwa 50 %-Masse, dann Abtupfen der Wimpern, dann Aufbringen von Komponente B in etwa 50 %-Masse) in einer Gesamtzeit von zwischen 10 und 20 Min. einwirken gelassen. Das Ergebnis war jeweils eine deutliche, permanente Färbung der Wimpern mit einem Farbton in Abhängigkeit vom verwendeten Extrakt (Catechine, Gallussäure, Gallate und Mischungen hievon) und seiner Konzentration in Komponente A und der Konzentration bzw. der aufgebrachten Menge an Silbernitrat in Komponente B.

## Patentansprüche

1. Gebrauchsfertige Färbezusammensetzung, enthaltend eine Komponente A umfassend mindestens ein Extrakt von Zaubernuss, Grüntee, dem Holz der Gerber-Akazie Acacia catechu, Granatapfel und Mischungen hiervon zusammen mit einer Komponente B umfassend ein Silbersalz in Form einer ammoniakalischen Lösung, wobei die gebrauchsfertige Färbezusammensetzung aus dem enthaltenen Extrakt mindestens 0,02 Gew.% mindestens einer Verbindung ausgewählt aus der Gruppe bestehend aus Catechinen, Gallussäure, Gallate und Mischungen hievon, enthält.

2. Färbezusammensetzung nach Anspruch 1, **dadurch gekennzeichnet, dass** sie in der Komponente A weiters mindestens eine Verbindung ausgewählt aus der Gruppe umfassend p-Phenylendiamin, p-Toluylendiamine, N-Phenyl-p-phenylendiamin, Hydroxyethyl-p-phenylendiamin, N,N'-Bis-hydroxyethyl-p-phenylendiamin, 2-Chloro-p-phenylendiamin, Triaminopyrimidin, Tetraaminopyrimidin, m-Aminophenol, p-Aminophenol, o-Aminophenol, Resorcinol, 2-Methylresorcinol, 4-Chlororesorcinol, 2,6-Diaminopyridin, 2,6-Dimethoxy-3,5-diaminopyridin, 2-Amino-3-hydroxypyridin, 2-Methyl-5-hydroxyethylaminophenol, NN'Bis-Hydroxyethyl-p-phenylendiamin 2,4-Diaminophenoxyethanol, 2-Amino-4-Hydroxyethylaminoanisolsulfat, 4-Amino-m-Cresol, 6-Amino-m-Cresol, Phenylmethylpyrazolon, 4-Amino-2-hydroxytoluol, p-Methylaminophenol, Hydroxybenzomorpholin, 1,3-bis-(2,4-Diaminophenoxy)propan, 6-Methoxy-2-methylamino-3-aminopyridin, 5-Amino-4-chloro-o-Cresol, 3-Amino-2,4-dichlorophenol, 1-Naphtol, 1,5-Naphtalendiol, 2,7-Naphtalendiol, 1-Hydroxyethyl-4,5-diaminopyrazolsulfat, 2,2'-Methylenbis-4-aminophenol, 2-Methyl-1-naphtol, 1-Acetoxy-2-methoxynaphtalen und Mischungen hievon enthält.

3. Färbezusammensetzung nach einem der Ansprüche 1 oder 2, **dadurch gekennzeichnet, dass** sie einen pH im Bereich von etwa 5 bis 9, vorzugsweise zwischen etwa 6 und 8, aufweist.

4. Färbezusammensetzung nach einem der Ansprüche 1 bis 3, **dadurch gekennzeichnet, dass** sie weiters Zusätze ausgewählt aus der Gruppe bestehend aus Pigmenten, Tensiden, Verdickungsmittel, Emulsionsgrundlagen, Lösungsmittel, Konservierungsmittel, Natriumsulfit und Mischungen hievon enthält.

5. Färbezusammensetzung nach einem der Ansprüche 1 bis 4, **dadurch gekennzeichnet, dass** sie als Komponente A Extrakte von Zaubernuss, Grüntee, Akazie und Granatapfel sowie Mischungen hievon, enthaltend Gallate, vorzugsweise in einer Menge von 0,1 bis 5 Gew.%, besonders bevorzugt 0,3 bis 3 Gew.% umfasst, wobei die Angaben an Gew.% sich auf die Komponente A beziehen.

6. Erzeugnis zur zeitlich abgestuften Anwendung, umfassend zwei Komponenten A und B, wobei Komponente A Extrakte von Zaubernuss, Grüntee, Akazie und Granatapfel sowie Mischungen hievon, enthaltend mindestens 0,02 Gew.% Gallate, vorzugsweise in einer Menge von 0,1 bis 5 Gew.%, besonders bevorzugt 0,3 bis 3 Gew.% umfasst, wobei die Angaben an Gew.% sich auf Komponente A beziehen, und Komponente B eine Zubereitung enthaltend Silbernitrat in einer basischen ammoniakalischen Lösung mit einem pH-Wert von zwischen 7 und 10, bevorzugt 8 bis 9, umfasst.

7. Erzeugnis nach Anspruch 6, **dadurch gekennzeichnet, dass** die beiden Komponenten A und B in Form einer wässrigen Lösung, einer Emulsion, oder eines Gels vorliegen bzw. Komponente A in Pulverform vorliegen und die Komponenten A und B gegebenenfalls zur Stabilisierung noch Verdickungsmittel enthalten.

8. Erzeugnis nach einem der Ansprüche 6 oder 7, **dadurch gekennzeichnet, dass** die Komponente B Silbernitrat in einer Menge von 2 bis 3 Gew.%, bevorzugt 1,8 bis 2,2 Gew.%, enthält, wobei die Angaben an Gew.% sich auf Komponente B beziehen.

## Claims

1. Ready-to-use dye composition, containing a component A comprising at least one extract of witch hazel, green tea, the wood of *Acacia catechu,* pomegranate and mixtures thereof, together with a component B comprising silver salt in the form of an ammoniac solution, wherein the ready-to-use dye composition containing the extract contains at least 0.02 wt.% at least one compound selected from the group of catechins, gallic acid, gallates and mixtures thereof.

2. Dye composition according to claim 1, **characterised in that** the composition further contains, in component A, at least one compound selected from the group of p-phenylenediamine, p-toluenediamine, N-phenyl-p-phenylenediamine, hydroxyethyl-p-phenylenediamine, N,N'-bis-hydroxyethyl-p-phenylenediamine, 2-chloro-p-phenylenediamine, triaminopyrimidine, tetraminopyrimidine, m-aminophenol, p-aminophenol, o-aminophenol, resorcinol, 2-methylresorcinol, 4-chlororesorcinol, 2,6-diaminopyridine, 2,6-dimethoxy-3,5-diaminopyridine, 2-amino-3-hydroxypyridine, 2-methyl-5-hydroxyethylaminophenol, N,N'-bis-hydroxyethyl-p-phenylenediamine, 2, 4-diaminophenoxyethanol, 2-amino-4-hydroxyethylaminoanisole sulphate, 4-amino-m-cresol, 6-amino-m-cresol, phenyl methyl pyrazolone, 4-amino-2-hydroxytoluene, p-methylaminophenol, hydroxybenzo-morpholine, 1,3-bis-(2,4-diaminophenoxy)propane, 6-methoxy-2-methylamino-3-aminopyridine, 5-amino-4-chloro-o-cresol, 3-amino-2,4-dichlorophenol, 1-naphthol, 1,5-naphthalenediole, 2,7-naphthalemediol, 1-hydroxyethyl-4,5-diamino pyrazole sulphate, 2,2'-methylenebis-4-aminophenol, 2-methyl-1-naphthol, 1-acetoxy-2-methoxynaphthalene and mixtures thereof.

3. Dye composition according to either claim 1 or claim 2, **characterised in that** it has a pH in the range of approximately 5 to 9, preferably between approximately 6 and 8.

4. Dye composition according to any of claims 1 to 3, **characterised in that** it further contains additives selected from the group of pigments, surfactants, thickening agents, emulsion bases, solvents, preservatives, sodium sulphite and mixtures thereof.

5. Dye composition according to any of claims 1 to 4, **characterised in that** it comprises extracts of witch hazel, green tea, acacia and pomegranate as well as mixtures thereof, containing gallates, preferably in an amount of 0.1 to 5 wt.%, particularly preferably 0.3 to 3 wt.%, as component A, the amounts in wt.% relating to component A.

6. Product for temporally gradual use, comprising two components A and B, wherein component A contains extracts of witch hazel, green tea, acacia and pomegranate as well as mixtures thereof, containing at least 0.02 wt.% gallates, preferably in an amount of 0.1 to 5 wt.%, particularly preferably 0.3 to 3 wt.%, wherein the amounts in wt.% relate to component A, and component B comprises a preparation contained silver nitrate in a basic ammoniac solution having a pH of between 7 and 10, preferably 8 to 9.

7. Product according to claim 6, **characterised in that** the two components A and B are in the form of an aqueous solution, an emulsion or a gel, or component A is in the form of a powder, and components A and B optionally additionally contain thickening agents for stabilisation.

8. Product according to either claim 6 or claim 7, **characterised in that** component B contains silver nitrate in an amount of 2 to 3 wt.%, preferably 1.8 to 2.2 wt.%, the amounts in wt.% relating to component B.

## Revendications

1. Composition tinctoriale prête à l'emploi, contenant un composant A comprenant au moins un extrait d'hamamélis, de thé vert, du bois de l'acacia à cachou Acacia catechu, de grenade et de mélanges de ceux-ci, ainsi qu'un composant B comprenant un sel d'argent sous forme d'une solution ammoniacale, la composition tinctoriale prête à l'emploi contenant, de l'extrait contenu, au moins 0,02 % en poids d'au moins un composé choisi dans le groupe consistant en les catéchines, l'acide gallique, les gallates et les mélanges de ceux-ci.

2. Composition tinctoriale selon la revendication 1, **caractérisée en ce qu'**elle contient en outre au moins un composant A choisi dans le groupe comprenant la p-phénylènediamine, les p-toluylènediamines, la N-phényl-p-phénylènediamine, l'hydroxyéthyl-p-phénylènediamine, la N,N'-bis-hydroxyéthyl-p-phénylènediamine, la 2-chloro-p-phénylènediamine, la triaminopyrimidine, la tétraaminopyrimidine, le m-aminophénol, le p-aminophénol, l'o-aminophénol, le résorcinol, le 2-méthylrésorcinol, le 4-chlororésorcinol, la 2,6-diaminopyridine, la 2,6-diméthoxy-3,5-diaminopyridine, la 2-amino-3-hydroxypyridine, le 2-méthyl-5-hydroxyéthylaminophénol, la N,N'-bis-hydroxyéthyl-p-phénylènediamine, le 2,4-diaminophénoxyéthanol, le sulfate de 2-amino-4-hydroxyéthylaminoanisole, le 4-amino-m-crésol, le 6-amino-m-crésol, la phénylméthylpyrazolone, le 4-amino-2-hydroxytoluène, le p-méthylaminophénol, l'hydroxybenzomorpholine, le 1,3-bis-(2,4-diaminophénoxy)propane, la 6-méthoxy-2-méthylamino-3-aminopyridine, le 5-amino-4-chloro-o-crésol, le 3-amino-2,4-dichlorophénol, le 1-naphtol, le 1,5-naphtalènediol, le 2,7-naphtalènediol, le sulfate de 1-hydroxyéthyl-4,5-diaminopyrazole, le 2,2'-méthylènebis-4-aminophénol, le 2-méthyl-1-naphtaol, le 1-acétoxy-2-méthoxynaphtalène et les mélanges de ceux-ci.

3. Composition tinctoriale selon l'une des revendications 1 ou 2, **caractérisée en ce qu'**elle présente un pH compris dans la plage d'environ 5 à environ 9, de préférence compris entre environ 6 et 8.

4. Composition tinctoriale selon l'une des revendications 1 à 3, **caractérisée en ce qu'**elle contient en outre des additifs choisis dans le groupe constitué par les pigments, les tensioactifs, les épaississants, les bases d'émulsions, les solvants, les conservateurs, le sulfite de sodium et les mélanges de ceux-ci.

5. Composition tinctoriale selon l'une des revendications 1 à 4, **caractérisée en ce qu'**elle comprend en tant que composant A des extraits d'hamamélis, de thé vert, d'acacia et de grenade, ainsi que des mélanges de ceux-ci, contenant des gallates, de préférence en une quantité de 0,1 à 5 % en poids, d'une manière particulièrement préférée de 0,3 à 3 % en poids, les indications de pourcentage en poids étant rapportées au composant A.

6. Produit destiné à une utilisation étagée dans le temps, comprenant deux composants A et B, le composant A comprenant des extraits d'hamamélis, de thé vert, d'acacia et de grenade, ainsi que des mélanges de ceux-ci, contenant au moins 0,02 % en poids de gallates, de préférence en une quantité de 0,1 à 5 % en poids, d'une manière particulièrement préférée de 0,3 à 3 % en poids, les indications de pourcentage en poids étant rapportées au composant A, et le composant B comprenant une préparation contenant du nitrate d'argent dans une solution ammoniacale basique présentant un pH compris entre 7 et 10, de préférence de 8 à 9.

7. Produit selon la revendication 6, **caractérisé en ce que** les deux composants A et B se présentent sous forme d'une solution aqueuse, d'une émulsion ou d'un gel, ou le composant A se présente sous forme d'une poudre et les composants A et B contiennent éventuellement encore, à des fins de stabilisation, des épaississants.

8. Produit selon l'une des revendications 6 ou 7, **caractérisé en ce que** le composant B contient du nitrate d'argent en une quantité de 2 à 3 % en poids, de préférence de 1,8 à 2,2 % en poids, les indications de pourcentage en poids étant rapportées au composant B.
